# EUROPEAN PATENT SPECIFICATION

(11) **EP 3 506 876 B1**
(45) Date of publication and mention of the grant of the patent: **03.03.2021**
(21) Application number: 17765073.6
(22) Date of filing: 31.08.2017
(51) Int. Cl.: A61K 8/365, A61K 8/37, A61Q 17/00, A61K 8/92, A61P 31/10, A61P 17/00

(54) **COMPOSITION FOR NAIL FUNGUS**
ZUSAMMENSETZUNGEN FÜR NAGELPILZ
COMPOSITION POUR LE TRAITEMENT DU CHAMPIGNON DES ONGLES

(30) Priority: 31.08.2016 DK PA201670670; 09.09.2016 DK PA201670693
(43) Date of publication of application: 10.07.2019
(73) Proprietor: Unigroup ApS, 2800 Lyngby (DK)
(72) Inventor: LICHT, Flemming, 2800 Kgs. Lyngby (DK)
(74) Representative: Holme Patent A/S
(86) International application number: PCT/DK2017/050278
(87) International publication number: WO 2018/041319

(56) References cited:
- EP-A2- 2 692 332
- WO-A1-2006/087359
- GB-A- 2 431 581
- US-A- 3 806 593
- US-A- 4 540 567
- US-A- 4 591 602
- US-B1- 6 740 326
- SINEGUB G A ET AL: "Treatment of fungal and viral diseases of skin and nails - by applying to affected areas ointment derived from vegetable oil ozonide(s) having peroxide number of 200-700", WPI / 2017 CLARIVATE ANALYTICS,, vol. 1994, no. 1, 15 March 1994 (1994-03-15), XP002941525, & RU 2 008 898 C1 (SUKOLIN GENNADIJ I [RU]; YAKOVLEV ALEKSEJ B [RU]; SINEGUB GEORGIJ A [R) 15 March 1994 (1994-03-15)
- V. TRAVAGLI ET AL: "Ozone and Ozonated Oils in Skin Diseases: A Review", MEDIATORS OF INFLAMMATION., vol. 2010, 1 January 2010 (2010-01-01), pages 1-9, XP055359108, GB ISSN: 0962-9351, DOI: 10.1155/2010/610418
- SILVIA MENÉNDEZ ET AL: "Therapeutic efficacy of topical OLEOZON in patients suffering from onychomycosis", MYCOSES, vol. 54, no. 5, 1 September 2011 (2011-09-01), pages e272-e277, XP055037320, ISSN: 0933-7407, DOI: 10.1111/j.1439-0507.2010.01898.x
- M. Cirlini ET AL: "Stability Studies of Ozonized Sunflower Oil and Enriched Cosmetics with a Dedicated Peroxide Value Determination", OZONE: SCIENCE AND ENGINEERING., vol. 34, no. 4, 1 July 2012 (2012-07-01), pages 293-299, XP055672766, US ISSN: 0191-9512, DOI: 10.1080/01919512.2012.692992

## Description

The present invention relates to a composition comprising ozonated oil. Further, the invention concerns a topical composition. The invention further concerns a composition for the treatment of nail fungus. In particular, the invention concerns a composition comprising ozonated oil for the treatment of nail fungus.

### Technical Background

The use of ozonated olive oil for toenail fungus has been described ("The Benefits of Ozonated Olive Oil for Toenail Fungus" by Dr. Edward Group).

The use of ozonized sunflower oil for treatment of onychomycosis has been described ("Therapeutic efficacy of topical OLEOZON in patients suffering from onychomycosis" by Menéndez S. et al, 2010).

RU2008898 C1 discloses the use of ozonised vegetable oils such as sunflower or olive oil for the treatment of skin or nail fungus.

The patent document US6740326 B1 (MEYER, H. et al.) relates to a water-free topical application composition for the treatment of nail diseases and nail care consisting essentially of one or more antimycotic substances; and one or more C1-C4-alkyl esters as carrier, wherein the carrier is a penetration promoting substance.

The article "Transonychial water loss in healthy and diseased nails" by Krönauer C et al. 2001 describes changes in transepidermal water loss (TEWL) and transonychial water loss (TOWL) from the skin and the nails in patients suffering from atopic eczema, psoriasis and onychomycosis compared to healthy individuals.

### Summary of the invention

There is a need for improved ozonated oil compositions. The present invention may provide a number of advantages.

Nail fungus is often associated with discoloration of the nail. The present invention may provide a whitening effect which counteracts such discoloration.

A composition of the invention may provide improved storage stability; in particular a color change of ozonated oils associated with long term storage of ozonated oils is minimized.

Ozonated oil experiences a rapid increase of peroxide and aldehyde levels at room temperature and high temperatures. A composition of the invention may provide improved storage stability.

A composition of the present invention may provide improved penetration into a nail, and may ensure complete penetration. This provides better interaction between the composition and any toenail and/or fingernail fungus, as well as decreased local concentration of ozone on the surface of the nail upon topical application. This may further improve the stability of the composition.

In addition a composition of the invention may decrease the odor of ozone, which may appear repugnant to users of known ozone containing compositions.

The treatment of nail fungus is often associated with a very long treatment time. A composition of the invention may provide a faster effect, meaning that a shorter treatment time will be necessary.

When applying a composition at the nail, the treated person will most often have to sit and wait for the composition to penetrate the nail. A composition of the invention may provide faster penetration from the nail surface through the nail. This faster penetration results in a faster removal from the nail surface, giving several advantages; socks can be worn immediately after application, and nail polish can be applied immediately after application of the composition.

It can be challenging to apply compositions with either very high or very low viscosity, leading to an unwanted loss of the composition and a bad user experience. A composition of the invention may provide a beneficial adjustment of the viscosity, leading to improved application of the composition, hence less unwanted loss and a more positive user experience.

The density of a composition varies with temperature and pressure. A composition of the invention may provide a beneficial adjustment of the density, leading to a more homogenous composition, and improved buoyancy, purity and packaging of the composition.

It has been speculated that the antimicrobial activity of ozonated oils increases in liquid environment with acidic pH ("Ozone therapy in periodontics", Gupta G. and Mansi B. 2012). A composition of the invention may provide a beneficial adjustment of the pH by lowering the pH value, hence increasing the antimicrobial activity of the ozonated oil.

Persons with slowly growing nails have an increased risk of having nail fungus and slowly growing nails take longer time to recover from nail fungus. A composition of the invention may accelerate the growth of the nail, leading to prevention and/or prophylaxis and/or faster curation from nail fungus.

Persons with thickened nails have an increased risk of having nail fungus and thickened nails take longer time to recover from nail fungus. A composition of the invention may help the nail regain normal thickness faster, leading to prevention and/or prophylaxis and/or faster curation from nail fungus.

Fungal tests are used to help detect and diagnose a fungal infection, to help guide treatment, and/or to monitor the effectiveness of treatment. A negative test after a person has been treated for a fungal infection means that the therapy has been successful. A composition of the invention may help reach a negative fungal test faster, ensuring that the source of the nail fungus is efficiently removed.

Compositions with ethyl lactate alone provide a limited saturation of the nail. A composition of the invention may provide a faster saturation of the nail, ensuring that the entire nail is saturated in a complete and fast way. Full saturation of the entire nail, including the nail bed and nail root, is important to protect the entire nail against nail fungus.

Nail fungus is a contagious disease, allowing spread within the nail, to other nails and even to the surrounding skin. Previous treatments for nail fungus only seek to cure the nail fungus. A composition of the invention may be capable of filling out all cavities in the nail, thereby preventing the fungi to dissipate in the nail and/or to the skin; in particular this will result in better protection of the nail and the skin from the fungi, decrease the probability of spread and/or decrease the probability of relapse.

A composition of the invention may allow simultaneous treatment of the nail and the skin. Previous treatments focus on treating either on or the other.

It could be speculated that the surface tension between the applied composition and the nail itself influences the treatment. A composition of the invention may provide an improved surface tension of the composition.

Increased TEWL is a well-recognized problem in skin conditions such as atopic eczema and psoriasis. It could be speculated that disrupted TEWL/TOWL balance is also a problem in nail conditions, such as nail fungus. A composition of the invention may normalize the TEWL/TOWL balance in the nail and/or in the skin.

According to an aspect the present invention concerns a composition comprising an ozonated oil and lactic acid and ethyl lactate.

According to another aspect the invention concerns a use of a composition of the invention for the treatment of nail fungus.

According to an aspect the invention concerns a use of a composition of the invention for preventing or counteracting discolored or thick nails, or alleviating discomfort associated with discolored or thick nails.

According to an aspect the invention concerns a use of a composition of the invention for the prevention or treatment of infections, such as bacterial, viral and/or fungal, (e.g. gingivitis, athletes foot, fungal skin infections, and/or herpes); skin conditions (e.g. eczema); and/or wounds (e.g. insect bites and/or stings, cuts, burns, leg ulcers, and/or bed sores).

### Detailed Disclosure

According to different embodiments, the present invention concerns the compositions and uses of the claims.

According to an embodiment, the invention concerns a composition comprising an ozonated oil; and lactic acid and ethyl lactate.

Ozone is a colorless gas of pungent odor. It can oxidize organic substances.

The term "ozonated oil" is also known as "ozonized oil", see e.g. "Ozonized vegetable oils and therapeutic properties: A review" (by Nathália R. de Almeida et al., Orbital, Elect. J. Chem., 4(4):313-326, 2012). Ozonated vegetable oils can be liquids or semisolids at room temperature. Ozonated sunflower oil (OLEOZON) purportedly has antimicrobial activity against bacteria, fungi and virus.

As an example, ozonated olive oil may be made with olive oil that is put through a process of "ozone injection", which is bubbling ozone into the liquid for an extended period of time. A preparation may be made by bubbling ozone in pure olive oil for at least 30 minutes in a cooled bath. Another option is to continue ozonation for two days until the pure olive oil solidifies. The article "Ozonized vegetable oils and therapeutic properties: A review" comprises further references, as well as methods of characterization of ozonated oils. The article "Ozone and Ozonated Oils in Skin Disesases: A review" (by V. Travagli, et al., Mediators of Inflammation, Vol. 2010, Article ID 610418) as well as "WFOT's Review on Evidence Based Ozone Therapy" (by the WFOT Scientific Advisory Committee 2015 (version 1)) provide additional information concerning the manufacture and characterization of ozonated oils. According to a preferred embodiment sun flower oil is used, and ozonation is continued in 6-8 hours.

Oils that have been successfully ozonated include ozonated olive oil, ozonated jojoba oil, ozonated sesame oil, ozonated peanut oil, ozonated coconut oil, and other oils. These ozonated oils are *inter alia* used for their antibacterial properties as well as their healing properties.

According to an embodiment the invention concerns the composition, wherein the total amount (w/w) of said ozonated oil is 1 - 90%, preferably 2 - 80%, more preferred 3 - 70%, preferably 4 - 60%, more preferred 5 - 50%, preferably 6 - 40%, more preferred 7 - 30%, preferably 8 - 20%, more preferred 9 - 15%, preferably about 10%.

It is speculated that ethyl lactate hydrolyses in situ, and the formed lactic acid acts with ozone products to provide improved antibacterial effect.

According to an embodiment the invention concerns the composition, wherein the total amount (w/w) of ethyl lactate, in the composition is 0.05 - 99.9%, preferably 1 - 99.5%, more preferred 10 - 99%, preferably 20 - 98%, more preferred 30 - 97%, preferably 40 - 96%, more preferred 50 - 95%, preferably 60 - 94%, more preferred 70 - 93%, preferably 75 - 92%, more preferred 80 - 90%, preferably 80 - 91 %, more preferred 81 - 89%, preferably 82.5 - 87.5%. According to an embodiment the invention concerns the composition, wherein the total amount (w/w) of lactic acid in the composition is 0.05 - 40%, preferably 0.1 - 25%, more preferred 0.2 - 15%, preferably 0.4 - 10%, more preferred 0.6 -5%, preferably 0.8 - 3%, more preferred 1 - 2.5%, preferably 1.2 -2%, more preferred about 1.5%.

According to an embodiment the invention concerns the composition, wherein said oil is plant oil. According to an embodiment the invention concerns the composition, wherein said oil is selected among the group consisting of the plant oils of Flax (Linseed), Hemp (C. sativa), Evening primrose, Safflower, Chia, Kukui (candle nut), Perilla, Grape seed, Pumpkin seed, Sunflower, Walnut, Soybean, Corn, Wheat germ, Rape (Canola), Sesame, Cotton seed, Rice bran, Beechnut, Sweet almond, Olive, Avocado, and mixtures of these. Further explanation and details may be found in "Ozonated Liquids in Dental Practice - A Review" by Dr Julian Holmes (April 2008, Part 4: The Chemistry of Ozone in Plant Oils).

According to an embodiment the invention concerns the composition, wherein said oil is selected among the group consisting of the plant oils of Hemp Oil, Canola (Rape) Oil, Sunflower Oil, Olive Oil, Avocado Oil, and mixtures of these.

According to an embodiment the invention concerns the composition, wherein said oil is selected among the group consisting of Flax (Linseed) Oil, Hemp Oil, Olive Oil, Sunflower Oil, and mixtures of these.

According to an embodiment the invention concerns the composition, wherein said oil is Sunflower Oil.

According to an embodiment the invention concerns the composition, further comprising:
c. A buffering agent to at least one C1-C4 alkyl ester, preferably said buffering agent is lactic acid.

While the term "buffering agent" is used, it is understood that said agent might not act as a buffering agent in a composition of the invention.

According to an embodiment the invention concerns the composition, wherein the total amount (w/w) of said buffering agent in the composition is 0.05 - 40%, preferably 0.1 - 25%, more preferred 0.2 - 15%, preferably 0.4 - 10%, more preferred 0.6 -5%, preferably 0.8 - 3%, more preferred 1 - 2.5%, preferably 1.2 -2%, more preferred about 1.5%.

According to an embodiment the invention concerns the composition, further comprising:
d. A fatty acid selected among the group consisting of medium-chain fatty acids (C₆-C₁₂), preferably an acid selected among the group consisting of caprylic, capric, caproic or lauric acids, preferably caprylic acid.

According to an embodiment the invention concerns the composition, wherein the total amount (w/w) of said fatty acid, preferably caprylic acid, in the composition is 0.1 - 10%, preferably 0.2 - 8%, more preferred 0.3 - 5%, preferably 0.4 - 4%, more preferred 0.5 - 3%, preferably 0.6 - 2%, more preferred 0.7 -1.5%, preferably 0.8 -1%, more preferred about 0.9%.

According to an embodiment the invention concerns the composition, further comprising:
e. A solvent selected among water, ethanol and isopropyl alcohol, preferably water.

According to an embodiment the invention concerns the composition, wherein the total amount (w/w) of said solvent, such as water, in the composition is 0- 90%, preferably 0.1 - 75%, more preferred 0.3 - 50%, preferably 0.5 - 25%, more preferred 1 - 15%, preferably 1.5 - 10%, more preferred 2 - 5%, preferably 2.5 - 4%, more preferred about 3%.

According to an embodiment the invention concerns the composition, further comprising:
f. A preservative or antioxidant, preferably selected among the group consisting of vitamin C, vitamin E, Rosmarinus officinalis extract, *Origanum vulgare* Leaf Extract, and mixtures of these.

According to an embodiment a preservative or antioxidant is preferably selected among the group consisting of vitamin C, vitamin E and extracts from the *Lamiaceae* family containing essential oils. The extracts are most preferably extracted from *Rosmarinus officinalis* (Ait-ouazzou, A. et al., "Chemical composition and antimicrobial activity of essential oils of Thymus algeriensis, Eucalyptus globulus and Rosmarinus officinalis from Morocco" 2643-2651 (2011)), *Origanum vulgare* Leaf (Zhang, X. et al., "Phenolic compounds from Origanum vulgare and their antioxidant and antiviral activities" FOOD Chem. 152, 300-306 (2014)), and mixtures of these both of which showing antimicrobial, antiviral and antifungal activities (Nolkemper, Silke, et al., Antiviral Effect of Aqueous Extracts from Species of the Lamiaceae Family against Herpes simplex Virus Type 1 and Type 2 in vitro" (2006); Delic, Dafina N., et al., "Antifungal Activity of Essential Oils of Origanum Vulgare and Rosmarinus Officinalis against Three Candida Albicans Strains" 203-211 (2013); Jnaid, Y. et al., "Antioxidant and antimicrobial activities of Origanum vulgare essential oil" IFRJ 23(4): 1706-1710 (2016)).

According to an embodiment the invention concerns the composition, wherein the total amount (w/w) of said preservative or antioxidant, in the composition is 0.01- 15%, preferably 0.02 - 10%, more preferred 0.03 - 5%, preferably 0.04 - 2%, more preferred 0.05 - 1.5%, preferably 0.06 - 1.0%, more preferred 0.07 - 0.5%, preferably 0.08 - 0.2%, more preferred 0.09 - 0.15%, preferably about 0.1%.

According to an embodiment the invention concerns the composition, comprising a mixture of Rosmarinus officinalis extract and *Origanum vulgare* Leaf Extract.

According to an embodiment the invention concerns the composition, wherein the total amount (w/w) of said mixture of Rosmarinus officinalis extract and *Origanum vulgare* Leaf Extract, in the composition is 0.01 - 15%, preferably 0.02 - 10%, more preferred 0.03 - 5%, preferably 0.04 - 2%, more preferred 0.05 - 1.5%, preferably 0.06 - 1.0%, more preferred 0.07 - 0.5%, preferably 0.08 - 0.2%, more preferred 0.09 - 0.15%, preferably about 0.1%.

According to an embodiment the invention concerns the composition, further comprising:
g. An emulsifier, preferably a polysorbate, more preferred polysorbate 20.

According to an embodiment the invention concerns the composition, wherein the total amount (w/w) of said emulsifier in the composition is 0.05 - 10 %, more preferred 0.1- 5%, preferably 0.1-4%, more preferred 0.2 - 3%, preferably 0.2 - 2%, more preferred 0.3 - 1%, preferably 0.4 - 0.7%, more preferred about 0.5%.

According to an embodiment the invention concerns the composition, further comprising:
h. An antioxidant soluble in lipids, preferably selected among the group of vitamin E and ascorbyl palmitate, more preferred ascorbyl palmitate.

According to an embodiment the invention concerns the composition, wherein the total amount (w/w) of said antioxidant, such as ascorbyl palmitate, in the composition is 0.1- 5%, preferably 0.2 - 2%, more preferred 0.3 - 1%, preferably 0.4 - 0.7%, more preferred about 0.5%.

According to an embodiment the invention concerns the composition, wherein said composition is selected among a liquid, a paste, a solid, a cream, an ointment, a nail lacquer, a spray, an oil, a gel, a nail-polish, a serum, an applicator pen, a brush, and a cotton swab.

According to an embodiment the invention concerns the composition, wherein said composition is selected among a liquid, a paste, a solid, a cream, an ointment, a nail lacquer and a spray.

According to an embodiment the invention concerns the composition, wherein said composition has a dosage form selected among a salve, an oil, a creme, a gel, a liquid, a nail-polish, a serum and a lacquer.

According to an embodiment the invention concerns the composition, wherein said composition has an application form selected among an applicator pen, a brush, a spray and a cotton swab.

According to an embodiment the invention concerns the composition for topical treatment.

According to an embodiment the invention concerns the composition for the treatment of an indication selected among nail fungus, Athlete's foot, toe and foot fungus, fungal skin infections; preferably nail fungus.

According to an embodiment the invention concerns the composition for the treatment of onychomycosis.

According to an embodiment the invention concerns a use of a composition of the invention for the treatment of nail fungus.

According to an embodiment the invention concerns a use of a composition of the invention for preventing or counteracting discolored or thick nails, or alleviating discomfort associated with discolored or thick nails.

According to an embodiment the invention concerns a use of a composition of the invention for the prevention or treatment of infections, such as bacterial, viral and/or fungal, (e.g. gingivitis, athletes foot, fungal skin infections, and/or herpes); skin conditions (e.g. eczema); and/or wounds (e.g. insect bites and/or stings, cuts, burns, leg ulcers, and/or bed sores).

According to an embodiment the invention concerns a use of a composition of the invention for the prevention or treatment of athletes foot and/or fungal skin infections.

According to an embodiment the invention concerns a use of a composition of the invention for the prevention or treatment of an indication selected among Acne, Athlete's Foot, Bacterial Infections, Bed Sores, Blackheads, Bruises, Carbuncles, Cold Sores, Cuts and Wounds (including bed sores), Dandruff, Dermatitis, Eczema, Diaper Rash, Fungal Infections, Fungal Skin Infections, Impetigo, Insect Bites, Fistulae, Gingivitis, Herpes Simplex, Hemorrhoids, Leg Ulcers, Shingles, Sunburn, Ringworm, Psoriasis, Wrinkles, Age Spots, Spider Bites, and Burns.

According to an embodiment the invention concerns a use of a composition of the invention for the prevention or treatment of an indication selected among Acne and Psoriasis.

According to an embodiment the invention concerns the composition providing improved storage stability by minimizing the color change of ozonated oils associated with long term storage.

According to an embodiment the invention concerns the composition that can be stored without color change of ozonated oils for 1 month, preferably 2 months, more preferred 4 months, preferably 8 months, more preferred 12 months, preferably 18 months, more preferred 24 months, preferably 30 months, more preferred 36 months, preferably 48 months, more preferred 60 months.

According to an embodiment the invention concerns the composition providing improved storage stability.

According to an embodiment the invention concerns the composition providing a faster effect, resulting in a shorter treatment time.

According to an embodiment the invention concerns the composition providing a treatment time of less than 24 months, preferably less than 18 months, more preferred less than 16 months, preferably less than 12 months, more preferred less than 6 months, preferably less than 5 months, more preferred less than 4 months, preferably less than 3 months, more preferred less than 2 months, preferably less than 1 month, more preferred less than 2 weeks.

According to an embodiment the invention concerns the composition providing faster penetration from the nail surface through the nail.

According to an embodiment the invention concerns the composition providing faster penetration from the nail surface through the nail, thereby allowing application of socks on a dry toenail surface within less than 15 minutes, preferably less than 10 minutes, more preferred less than 5 minutes, preferably less than 4 minutes, more preferred less than 3 minutes, preferably less than 2 minutes, more preferred less than 1 minute, preferably less than 30 seconds.

According to an embodiment the invention concerns the composition providing faster penetration from the nail surface through the nail, thereby allowing application of nail polish on a dry nail surface within less than 15 minutes, preferably less than 10 minutes, more preferred less than 5 minutes, preferably less than 4 minutes, more preferred less than 3 minutes, preferably less than 2 minutes, more preferred less than 1 minute, preferably less than 30 seconds.

According to an embodiment the invention concerns the composition providing a beneficial adjustment of the viscosity.

According to an embodiment the invention concerns a use of a composition of the invention for a beneficial adjustment of viscosity.

According to an embodiment the invention concerns the composition having a density between 0.500 - 2.000, preferably 0.600 - 1.500, more preferred 0.700 - 1.400, preferably 0.800 - 1.300, more preferred 0.900 - 1.200, preferably 0.950 - 1.100 g/ml.

According to an embodiment the invention concerns the composition providing a beneficial adjustment of density.

According to an embodiment the invention concerns a use of a composition of the invention for a beneficial adjustment of density.

According to an embodiment the invention concerns the composition having a pH between 0.50 - 5, preferably 0.60 - 4, more preferred 0.7 - 3.5, preferably 0.8 - 3, more preferred 0.9 - 2.5, preferably 1- 2, more preferred 1- 1.8, preferable 1.1- 1.7, more preferred around 1.6.

According to an embodiment the invention concerns the composition providing a beneficial adjustment of the pH by lowering a pH value.

According to an embodiment the invention concerns a use of a composition of the invention for lowering a pH value.

According to an embodiment the invention concerns the composition having a refractive index between 1- 3, preferably 1.1- 2.5, more preferred 1.2 - 2.0, preferably 1.3 - 1.5.

According to an embodiment the invention concerns the composition accelerating the growth of the nail.

According to an embodiment the invention concerns the composition helping the nail regain normal thickness faster.

According to an embodiment the invention concerns the composition helping the nail regain normal thickness in less than 12 months, preferably less than 10 months, more preferred less than 6 months, preferably less than 5 months, more preferred less than 4 months, preferably less than 3 months, more preferred less than 2 months, preferably less than 1 month, more preferred less than 2 weeks.

According to an embodiment the invention concerns the composition facilitating and/or aiding a negative fungal test to be reached faster.

According to an embodiment the invention concerns the composition allowing a negative fungal test to be reached faster.

According to an embodiment the invention concerns the composition allowing a negative fungal test to be reached in less than 24 months, preferably less than 18 months, more preferred less than 16 months, preferably less than 12 months, more preferred less than 6 months, preferably less than 5 months, more preferred less than 4 months, preferably less than 3 months, more preferred less than 2 months, preferably less than 1 month.

According to an embodiment the invention concerns the composition providing a faster saturation of the nail.

According to an embodiment the invention concerns the composition capable of filling out all cavities in the nail, thereby preventing the fungi to dissipate in the nail and/or to the skin.

According to an embodiment the invention concerns the composition allowing simultaneous treatment of the nail and the skin.

According to an embodiment the invention concerns the composition providing an improved surface tension of the composition.

According to an embodiment the invention concerns the composition reducing and/or normalizing the TEWL/TOWL ratio in the nail and/or in the skin.

The accompanying Examples are provided to explain rather than limit the present invention. It will be clear to the person skilled in the art that aspects, embodiments and claims of the present invention may be combined.

Unless otherwise mentioned, all percentages are in weight/weight. Unless otherwise mentioned, all measurements are conducted under standard conditions (ambient temperature and pressure).

### Examples

### Example 1

A composition comprising Ethyl Lactate (83%), Ozonated oil (10%), Lactic acid (1.5%), Caprylic acid (1%), aqua (3%), Vitamin E (0.5%), polysorbate 20 (0.5%), ascorbyl palmitate (0.4%), and organum vulgare leaf extract (0.1%) was provided by mixing of the ingredients.

### Example 2

A composition comprising Ethyl Lactate (87,5%), Ozonated oil (10%), Lactic acid (1.5%), Caprylic acid (0,9%) and Organum vulgare leaf extract (0,1%) was provided by mixing the ingredients.

Vitamin E (and often also Vitamin C) is commonly used to stabilize the ozonated oil, in order to prolong the shelf life of the oil. However, the presence of Vitamin E will lead to final composition having a tendency to turn yellow upon storage for some time. Surprisingly, this yellow color does not result when Origanum vulgare Leaf Extract is used instead of vitamin E.

### Example 3

A composition comprising Ethyl lactate (85.5%), Ozonated oil (9.8%), Ascorbyl Tetraisopalmitate (2%), Lactic acid (1.5%), Caprylic acid (0.9%), Origanum vulgare Leaf extract (0.1%), Tocopheryl acetate (0.1%), L-Ascorbyl Palmitate (0.1%) was provided by mixing of the ingredients.

### Example 4

Compositions of Example 1-3 may be used for the topical treatment of nail fungus.

### Example 5

Compositions of Example 1-3 may be applied on nails and/or on skin to prevent and/or treat nails fungus and/or athletes foot and/or fungal skin infections.

## Claims

1. A composition for the treatment of nail fungus, which composition comprises:
a. ozonated oil; and
b. lactic acid and ethyl lactate.

2. The composition according to claim 1, wherein the total amount (w/w) of said ozonated oil is 1 - 90%, preferably 2 - 80%, more preferred 3 - 70%, preferably 4 - 60%, more preferred 5 - 50%, preferably 6 - 40%, more preferred 7 - 30%, preferably 8 - 20%, more preferred 9 - 15%, preferably about 10%.

3. The composition according to any of the preceding claims, wherein the total amount (w/w) of ethyl lactate in the composition is 0.05 - 99.9%, preferably 1 - 99.5%, more preferred 10 - 99%, preferably 20 - 98%, more preferred 30 - 97%, preferably 40 - 96%, more preferred 50 - 95%, preferably 60 - 94%, more preferred 70 - 93%, preferably 75 - 92%, more preferred 80 - 90%, preferably 80 - 91 %, more preferred 81 - 89%, preferably 82.5 - 87.5%.

4. The composition according to any of the preceding claims, wherein the total amount (w/w) of lactic acid in the composition is 0.05 - 40%, preferably 0.1 - 25%, more preferred 0.2 - 15%, preferably 0.4 - 10%, more preferred 0.6 -5%, preferably 0.8 - 3%, more preferred 1 - 2.5%, preferably 1.2 -2%, more preferred about 1.5%.

5. The composition according to any of the preceding claims, wherein said oil is plant oil.

6. The composition according to any of the preceding claims, wherein said oil is selected among the group consisting of the plant oils of Flax (Linseed), Hemp (C. sativa), Evening primrose, Safflower, Chia, Kukui (candle nut), Perilla, Grape seed, Pumpkin seed, Sunflower, Walnut, Soybean, Corn, Wheat germ, Rape (Canola), Sesame, Cotton seed, Rice bran, Beechnut, Sweet almond, Olive, Avocado, and mixtures of these.

7. The composition according to any of the preceding claims, wherein the composition further comprises at least one ingredient selected among
c. A buffering agent to the ethyl lactate, preferably said buffering agent is lactic acid.
d. A fatty acid selected among the group consisting of medium-chain fatty acids (C₆-C₁₂), preferably an acid selected among the group consisting of caprylic, capric, caproic or lauric acids, preferably caprylic acid.
e. A solvent selected among water, ethanol and isopropyl alcohol, preferably water.
f. A preservative or antioxidant, preferably selected among the group consisting of vitamin C, vitamin E, Rosmarinus officinalis extract, *Origanum vulgare* Leaf Extract, and mixtures of these.
g. An emulsifier, preferably a polysorbate, more preferred polysorbate 20.
h. An antioxidant soluble in lipids, preferably selected among the group of vitamin E and ascorbyl palmitate, more preferred ascorbyl palmitate.

8. The composition according to any of the preceding claims, wherein said composition is selected among liquid, a paste, a solid, a cream, an ointment, a nail lacquer, a spray, an oil, a gel, a nail-polish, a serum, an applicator pen, a brush, and a cotton swab.

9. The composition according to any of the preceding claims for topical treatment.

10. The composition according to any of the preceding claims for the treatment of an indication selected among fungal skin infections, Athlete's foot, toe and foot fungus.

11. The composition according to any of the preceding claims for the treatment of nail fungus.

12. The composition according to any of the preceding claims for use in the treatment of onychomycosis.

13. The composition according to any of the preceding claims for use in preventing or counteracting discolored or thick nails, or alleviating discomfort associated with discolored or thick nails.

14. The composition according to any of the preceding claims for use in the prevention or treatment of infections, such as bacterial, viral and/or fungal, such as gingivitis, athletes foot, fungal skin infections and/or herpes; skin conditions such as eczema; and/or wounds such as insect bites and/or stings, cuts, burns, leg ulcers, and/or bed sores.

## Patentansprüche

1. Zusammensetzung für die Behandlung von Nagelpilz, wobei die Zusammensetzung Folgendes umfasst:
a. ozonisiertes Öl und
b. Milchsäure und Ethyllaktat.

2. Zusammensetzung nach Anspruch 1, wobei die Gesamtmenge (Gew./Gew.) des ozonisierten Öls 1 - 90 %, vorzugsweise 2 - 80 %, mehr bevorzugt 3 - 70 %, bevorzugt 4 - 60 %, mehr bevorzugt 5 - 50 %, vorzugsweise 6 - 40 %, mehr bevorzugt 7 - 30 %, vorzugsweise 8 - 20 %, mehr bevorzugt 9 - 15 %, vorzugsweise ungefähr 10 % beträgt.

3. Zusammensetzung nach einem der vorstehenden Ansprüche, wobei die Gesamtmenge (Gew./Gew.) des Ethyllaktats in der Zusammensetzung 0,05 - 99,9 %, vorzugsweise 1 - 99,5 %, mehr bevorzugt 10 - 99 %, vorzugsweise 20 - 98 %, mehr bevorzugt 30 - 97 %, vorzugsweise 40 - 96 %, mehr bevorzugt 50 - 95 %, vorzugsweise 60 - 94 %, mehr bevorzugt 70 - 93 %, vorzugsweise 75 - 92 %, mehr bevorzugt 80 - 90 %, vorzugsweise 80 - 91 %, mehr bevorzugt 81 - 89 %, vorzugsweise 82,5 - 87,5 % beträgt.

4. Zusammensetzung nach einem der vorstehenden Ansprüche, wobei die Gesamtmenge (Gew./Gew.) der Milchsäure in der Zusammensetzung 0,05 - 40 %, vorzugsweise 0,1 - 25 %, mehr bevorzugt 0,2 - 15 %, vorzugsweise 0,4 - 10 %, mehr bevorzugt 0,6 - 5 %, vorzugsweise 0,8 - 3 %, mehr bevorzugt 1 - 2,5 %, vorzugsweise 1,2 - 2 %, mehr bevorzugt ungefähr 1,5 % beträgt.

5. Zusammensetzung nach einem der vorstehenden Ansprüche, wobei das Öl Pflanzenöl ist.

6. Zusammensetzung nach einem der vorstehenden Ansprüche, wobei das Öl aus der Gruppe ausgewählt ist, die aus den Pflanzenölen von Flachs (Leinsamen), Hanf (C. sativa), Nachtkerze, Saflor, Chia, Kukui (Kukuinuss), Perilla, Traubenkern, Kürbiskern, Sonnenblume, Walnuss, Sojabohne, Mais, Weizenkeim, Raps (Canola), Sesam, Baumwollsamen, Reiskleie, Buchecker, Süßmandel, Olive, Avocado und Mischungen davon besteht.

7. Zusammensetzung nach einem der vorstehenden Ansprüche, wobei die Zusammensetzung ferner mindestens einen Bestandteil umfasst, der aus Folgenden ausgewählt ist:
c. einem Puffermittel für das Ethyllaktat, wobei das Puffermittel vorzugsweise Milchsäure ist;
d. einer Fettsäure, die aus der Gruppe ausgewählt ist, die aus mittelkettigen Fettsäuren (C₆ - C₁₂) besteht, vorzugsweise einer Säure, die aus der Gruppe ausgewählt ist, die aus Capryl-, Caprin-, Capron- oder Laurinsäure, vorzugsweise Caprylsäure besteht;
e. einem Lösemittel, ausgewählt aus Wasser, Ethanol und Isopropylalkohol, vorzugsweise Wasser;
f. einem Konservierungsmittel oder Antioxidationsmittel, vorzugsweise ausgewählt aus der Gruppe, die aus Vitamin C, Vitamin E, Rosmarinus officinalis-Extrakt, *Origanum* vulgare-Blattextrakt und Mischungen von ihnen besteht;
g. einem Emulgator, vorzugsweise einem Polysorbat, mehr bevorzugt Polysorbat 20;
h. einem in Lipiden lösbaren Antioxidationsmittel, vorzugsweise ausgewählt aus der Gruppe von Vitamin E und Ascorbinsäurepalmitat, mehr bevorzugt Ascorbinsäurepalmitat.

8. Zusammensetzung nach einem der vorstehenden Ansprüche, wobei die Zusammensetzung aus Flüssigkeit, einer Paste, einem Feststoff, einer Creme, einer Salbe, einer Nagelpolitur, einem Spray, einem Öl, einem Gel, einem Nagellack, einem Serum, einem Applikatorstift, einer Bürste und einem Wattestäbchen ausgewählt ist.

9. Zusammensetzung nach einem der vorstehenden Ansprüche für topische Behandlung.

10. Zusammensetzung nach einem der vorstehenden Ansprüche für die Behandlung einer Indikation, die aus fungalen Hautinfektionen, Athletenfuß, Zehen- und Fußpilz ausgewählt ist.

11. Zusammensetzung nach einem der vorstehenden Ansprüche für die Behandlung von Nagelpilz.

12. Zusammensetzung nach einem der vorstehenden Ansprüche für die Behandlung von Onychomykose.

13. Zusammensetzung nach einem der vorstehenden Ansprüche zur Anwendung bei der Verhinderung oder Bekämpfung von verfärbten oder dicken Nägeln oder Linderung von Beschwerden im Zusammenhang mit verfärbten oder dicken Nägeln.

14. Zusammensetzung nach einem der vorstehenden Ansprüche zur Anwendung bei der Verhinderung oder Behandlung von Infektionen wie bakteriellen, viralen und/oder fungalen Infektionen wie zum Beispiel Zahnfleischentzündung, Athletenfuß, fungalen Hautentzündungen und/oder Herpes; Hautbeschwerden wie Ekzemen; und/oder Wunden wie Insektenbisse und/oder -stiche, Schnittwunden, Verbrennungen, Beingeschwüren und/oder Wundliegen.

## Revendications

1. Composition destinée au traitement d'une mycose des ongles, ladite composition comprenant :
a. une huile ozonisée ; et
b. de l'acide lactique et du lactate d'éthyle.

2. Composition selon la revendication 1, dans laquelle la quantité totale (en poids/poids) de ladite huile ozonisée s'élève de 1 à 90 %, de préférence de 2 à 80 %, de manière plus préférée de 3 à 70 %, de préférence de 4 à 60 %, de manière plus préférée de 5 à 50 %, de préférence de 6 à 40 %, de manière plus préférée de 7 à 30 %, de préférence de 8 à 20 %, de manière plus préférée de 9 à 15 %, de préférence s'élève à environ 10 %.

3. Composition selon l'une quelconque des revendications précédentes, dans laquelle la quantité totale (en poids/poids) du lactate d'éthyle dans la composition s'élève de 0,05 à 99,9 %, de préférence de 1 à 99,5 %, de manière plus préférée de 10 à 99 %, de préférence de 20 à 98 %, de manière plus préférée de 30 à 97 %, de préférence de 40 à 96 %, de manière plus préférée de 50 à 95 %, de préférence de 60 à 94 %, de manière plus préférée de 70 à 93 %, de préférence de 75 à 92 %, de manière plus préférée de 80 à 90 %, de préférence de 80 à 91 %, de manière plus préférée de 81 à 89 %, de préférence de 82,5 à 87,5 %.

4. Composition selon l'une quelconque des revendications précédentes, dans laquelle la quantité totale (en poids/poids) de l'acide lactique dans la composition s'élève de 0,05 à 40 %, de préférence de 0,1 à 25 %, de manière plus préférée de 0,2 à 15 %, de préférence de 0,4 à 10 %, de manière plus préférée de 0,6 à 5 %, de préférence de 0,8 à 3 %, de manière plus préférée de 1 à 2,5 %, de préférence de 1,2 à 2 %, de manière plus préférée s'élève à environ 1,5 %.

5. Composition selon l'une quelconque des revendications précédentes, dans laquelle ladite huile représente une huile végétale.

6. Composition selon l'une quelconque des revendications précédentes, dans laquelle ladite huile est choisie parmi le groupe constitué par les huiles végétales de lin (graines de lin), de chanvre (C. sativa), d'onagre, de carthame, de chia, de kukui (de noix de bancoul), de périlla, de pépins de raisin, de graines de courge, de tournesol, de noix, de soja, de maïs, de germe de blé, de colza (canola), de sésame, de noyaux de coton, de son de riz, de faine, d'amande douce, d'olive, d'avocat, et par des mélanges des huiles précitées.

7. Composition selon l'une quelconque des revendications précédentes, dans laquelle la composition comprend en outre au moins un ingrédient qui est choisi parmi :
c. un agent faisant tampon par rapport au lactate d'éthyle, de préférence ledit agent faisant tampon représente l'acide lactique ;
d. un acide gras qui est choisi parmi le groupe constitué par des acides gras à chaînes moyennes (en C₆-C₁₂), de préférence un acide qui est choisi parmi le groupe constitué par l'acide caprylique, l'acide caprique, l'acide caproïque ou l'acide laurique, de préférence l'acide caprylique ;
e. un solvant qui est choisi parmi l'eau, l'éthanol et l'alcool isopropylique, de préférence l'eau ;
f. un conservateur ou un antioxydant, qui est choisi de préférence parmi le groupe constitué par la vitamine C, la vitamine E, un extrait de Rosmarinus officinalis, un extrait de feuilles de *Origanum vulgare,* ainsi que des mélanges des éléments précités ;
g. un émulsifiant, de préférence un polysorbate, de manière plus préférée le polysorbate 20 ;
h. un antioxydant soluble dans des lipides, qui est choisi préférence parmi le groupe constitué par la vitamine E et par le palmitate d'ascorbyle, de manière plus préférée le palmitate d'ascorbyle.

8. Composition selon l'une quelconque des revendications précédentes, dans laquelle ladite composition est choisie parmi un liquide, une pâte, un produit solide, une crème, un onguent, une laque à ongles, un spray, une huile, un gel, un vernis à ongles, un sérum, un stylo applicateur, une brosse et un coton-tige.

9. Composition selon l'une quelconque des revendications précédentes, destinée à un traitement local.

10. Composition selon l'une quelconque des revendications précédentes, destinée au traitement d'une indication qui est choisie parmi des infections fongiques de la peau, une mycose, des champignons aux orteils et aux pieds.

11. Composition selon l'une quelconque des revendications précédentes, destinée au traitement d'une mycose des ongles.

12. Composition selon l'une quelconque des revendications précédentes, pour son utilisation dans le traitement d'une onychomycose.

13. Composition selon l'une quelconque des revendications précédentes, pour son utilisation dans la prévention ou la lutte contre des ongles décolorés ou épais, ou dans le soulagement d'un inconfort lié à des ongles décolorés ou épais.

14. Composition selon l'une quelconque des revendications précédentes, pour son utilisation dans la prévention ou le traitement d'infections telles que des infections bactériennes, virales et/ou fongiques telles que la gingivite, une mycose, des infections fongiques de la peau et/ou de l'herpès; des affections cutanées telles que l'eczéma ; et/ou des blessures telles que des morsures et/ou des piqûres d'insectes, des coupures, des brûlures, des ulcères de la jambe et/ou des escarres.
